Europäisches Patentamt

European Patent Office

Office européen des brevets

(11) Veröffentlichungsnummer: **0 406 395 B1**

(12) **EUROPÄISCHE PATENTSCHRIFT**

(45) Veröffentlichungstag der Patentschrift: **13.04.94**

(21) Anmeldenummer: **90902203.0**

(22) Anmeldetag: **17.01.90**

(86) Internationale Anmeldenummer:
**PCT/EP90/00087**

(87) Internationale Veröffentlichungsnummer:
**WO 90/08197 (26.07.90 90/17)**

(51) Int. Cl.5: **C12Q 1/68**, C07K 15/28,
C07K 13/00, G01N 33/569

Die Akte enthält technische Angaben, die nach
dem Eingang der Anmeldung eingereicht wurden
und die nicht in dieser Patentschrift enthalten sind.

(54) **VERFAHREN ZUR BESTIMMUNG VON PATHOGENEN LISTERIA-BAKTERIEN.**

(30) Priorität: **19.01.89 DE 3901397**
**03.03.89 DE 3906832**

(43) Veröffentlichungstag der Anmeldung:
**09.01.91 Patentblatt 91/02**

(45) Bekanntmachung des Hinweises auf die
Patenterteilung:
**13.04.94 Patentblatt 94/15**

(84) Benannte Vertragsstaaten:
**AT BE CH DE DK ES FR GB IT LI LU NL SE**

(56) Entgegenhaltungen:
**EP-A- 314 294**
**WO-A-89/06699**
**FR-A- 2 616 804**

**MOLECULAR CLONING, 1989; J. SAMBROOK
et al., pp. 9.47-9.51, 11.45-11.49&NUM;**

(73) Patentinhaber: **BOEHRINGER MANNHEIM
GMBH**

**D-68298 Mannheim(DE)**

(72) Erfinder: **CHAKRABORTY, Trinad
Friedrichstr. 12
D-8700 Würzburg(DE)**
Erfinder: **GOEBEL, Werner
Ravensburgstr. 2B
D-8707 Veitshöchheim(DE)**
Erfinder: **NOTERMANS, Servatius, Hubertus,
Wilhelmus
Obrechtlaan 17
NL-3723 KA Bilthoven(NL)**

**Beschreibung**

Die Erfindung betrifft ein Verfahren zur Bestimmung von pathogenen Listeria-Bakterien, eine Nachweisnucleinsäure (probe) sowie ein Protein, welches zur Herstellung von Antikörpern gegen pathogene Listeria-Bakterien geeignet ist.

Listerien sind eine heterogene Gruppe grampositiver Bakterien und bestehen im wesentlichen aus den Spezies Listeria monocytogenes, L. innocua, L. welshimeri, L. seeligeri, L. ivanovii, L. grayi und L. murrayi. Davon sind lediglich zwei Spezies pathogen, nämlich L. monocytogenes für Menschen und Tiere und L. ivanovii für Tiere. Die Listeriose äußert sich beim Menschen üblicherweise in einer bakteriellen Meningitis und Septikämie sowie bei Schwangeren in Fehl- und Totgeburten. Bei Schafen und Rindern äußert sich die Listeriose in Fehlgeburt, Enzephalitis, Septikämie und Mastitis (N. Engl. J. Med. 308 (1983), 203-206), J. Infec. 15 (1987), 165-168, Linnan, M.J. et al., An investigation of listeriosis in Southern California 1985, in Courtieu, A.L. et al.,(eds) Listeriose, Listeria, Listeriosis 1985-1986, University of Nantes).

In jüngster Zeit wurde eine Häufung von Listeriose-Erkrankungen beim Menschen beobachtet, als deren Ursache die Kontamination von Milch und Käse, vor allem von Weichkäsesorten,durch Listeria-Bakterien als Ursache angesehen wird. Demzufolge ist eine Überprüfung von Lebensmitteln auf Listeria-Kontamination wichtig und in USA bereits für Käse vorgeschrieben.

Aus Int. J. Food Microbiol. 4 (1987), 249-256 ist ein Verfahren zur Bestimmung von Listeria monocytogenes bekannt, bei dem der Mikroorganismus selektiv in flüssigem Medium bei 4°C angereichert, auf speziellen Agarböden gezüchtet und anschließend durch eine Vielzahl biochemischer Tests bio- und serotypisiert wird. Dieses Verfahren ist sehr arbeits- und zeitaufwendig und es dauert 14 Tage und länger bis das Endergebnis vorliegt. Außerdem ist die Beurteilung nur sehr schwierig durchführbar und nicht für Routineuntersuchungen geeignet.

Aus Appl. Environ. Microbiol. 53 (1987), 2256-2259 ist ein Verfahren zur Bestimmung von Listeria-Bakterien bekannt, welches durch colony-Hybridisierung mit radioaktiv markierten DNA-probes durchgeführt wird. Die colony-Hybridisierung wäre an sich ein geeignetes Verfahren für eine schnelle und einfache Analyse der Kontamination von Lebensmitteln durch Listeria-Bakterien. Dazu ist jedoch eine Nachweis-Nucleinsäure (probe) erforderlich, welche mit allen pathogenen Listeria-Bakterien (L. monocytogenes und L. ivanovii), nicht jedoch mit den anderen Listeria-Bakterien hybridisiert. Bisher bekannte DNA-probes (Listeriolysin O, β-Listeriolysin; vgl. Infection and Immunity 55 (1987), 3225-3227 und Applied and Environmental, Microbiology 53 (1987), 2256-2259) sind zu unspezifisch und ergeben demzufolge eine nicht akzeptable Anzahl falsch-positiver und falsch-negativer Ergebnisse.

Aufgabe der vorliegenden Erfindung ist es demnach, eine Nucleinsäure bereitzustellen, mit welcher spezifisch Hybridisierungstests auf pathogene Listeria-Bakterien möglich sind sowie hiervon abgeleitete Proteine, welches als Immunogene zur Herstellung von Antikörpern gegen pathogene Listeria-Bakterien geeignet sind.

Diese Aufgabe wird durch eine Nucleinsäure gelöst, die bei den Hybridisierungsbedingungen 5 - 6 x SSC und 42 - 60°C und den Waschbedingungen 0.1 - 0.5 x SSC bei 65° bis 75°C mit dem 2,5 kb großen KpnI-BamHI Fragment aus dem Plasmid pLM63 (DSM 5220) hybridisiert. Vorzugsweise ist die Nucleinsäure mindestens 27 Nucleotide lang. Ebenfalls bevorzugt ist eine Nucleinsäure, welche unter diesen Bedingungen mit der in dem oben genannten Fragment enthaltenen Nucleinsäuresequenz gemäß Tabelle III hybridisiert. Ebenfalls bevorzugt ist eine Nucleinsäure, welche unter diesen Bedingungen mit der Nucleinsäure entsprechend Nucleotid 822 - 1890 aus Tabelle III hybridisiert. Besonders bevorzugt wird die Nucleinsäure der Sequenz nach Tabelle III, ein Fragment dieser Sequenz von Nucleotid 1 - 714, 822 - 1890, 935 bis 1244 von 1158 bis 1185 oder von 1209 bis 1718 oder ein mindestens 27 Nucleotide langes Fragment der Sequenz nach Tabelle III verwendet. Die maximale Länge der Probe ist unkritisch. Üblicherweise sollte sie jedoch die Länge des pLM63-Fragments, also 2,5 kb, nicht wesentlich überschreiten.

Unter Nucleinsäuren im Sinne der Erfindung sind Oligodesoxyribonukleoside oder die entsprechenden Oligoribonucleoside sowie deren zur Hybridisierung geeignete Derivate zu verstehen.

Der Listeria-Nachweis erfolgt nach den dem Fachmann geläufigen molekularbiologischen Techniken der DNA/DNA, RNA/RNA bzw. RNA/DNA-Hybridisierung zum Nachweis homologer Nucleinsäuresequenzen. Dazu werden üblicherweise die Methoden der colony- oder plaque-Hybridisierung und blotting-Verfahren (z. B. Southern blot und dot blot) verwendet. Weitere bekannt Verfahren sind in USP 4 358 535, Gene 21 (1983) 7785, EP-B 130515, EP-B 70685, EP-B 70687 und EP-A 238332 beschrieben.

Die Markierung der probe erfolgt beispielsweise mit radioaktiv derivatisierten Desoxyribonucleosidtriphosphaten,nichtradioaktiv mit Biotin, Avidin, Streptavidin, einem Fluoreszenzfarbstoff oder einem Hapten. In letzterem Fall erfolgt der Nachweis des Hybridisierungsprodukts durch Bestimmung der enzymatischen Aktivität des Markerenzyms in einem Anti-Hapten-Antikörper-Enzym-Konjugat Über gekoppelte Farbstoffsy-

EP 0 406 395 B1

steme. Weitere Verfahren sind beispielsweise in der DE-A 38 13 278 und der DE-A 38 00 644 beschrieben. Die Substanz, mit der die probe markiert ist, wird dort auch als Reportergruppe bezeichnet.

Die Durchführung des erfindungsgemäßen Verfahrens erfolgt vorzugsweise dadurch, daß die erfindungsgemäßen Nucleinsäureprobe radioaktiv, mit Biotin, Streptavidin, Avidin oder mit einem Hapten markiert und anschließend Hybridisierung und Bestimmung der Markierung durchgeführt wird.

Zur Markierung der erfindungsgemäßen probes können verschiedene Methoden angewendet werden, wie sie beispielsweise in Molecular Cloning, Maniatis et al (1982), Cold Spring Habor Laboratory, Cold Spring Habor, New York, beschrieben sind. Der Einbau vom Biotin in Nucleinsäuren ist beispielsweise in PNAS USA 78 (1981) 6633 und PNAS USA 79 (1982) 7331 beschrieben. Eine weitere Methode zum Einbau von Reportergruppen ist in EP-A 292128 beschrieben.

Die radioaktive Markierung kann nach den bekannten Verfahren durchgeführt werden. Der Einbau eines Haptens kann beispielsweise enzymatisch, chemisch oder photochemisch erfolgen. Die Herstellung der Oligonucleotid-probes kann beispielsweise nach der "Random-primed" Methode (Anal. Biochem. 132 (1983) 6) der "Specific-primed" Methode, der "Reversen transcriptions" Methode (Stelow, J.K. und Holländer A. eds, Genetic Engineering, Plenum Press New York and London, Vol. 1, Seite 1) nach der "Fill in" der "Nick-Translation" Methode (J. Mol. Biol. 113 (1977) 237) der "Tailing" Methode, der "Transcriptions" Methode (J. Mol. Biol. 166 (1983) 477), der photochemischen Methode (Nucl. Acids Ris. 13 (1985) 745 - 761) oder chemisch durchgeführt werden. Diese Methoden sind beispielsweise in der DE-A 38 13 278 näher beschrieben.

Das zu untersuchende Material (sample) wird vor Durchführung der Bestimmung in üblicher Weise aufbereitet. Vorteilhaft wird dabei die RNA und/oder DNA aus der sample nach bekannten Verfahren isoliert (vgl. z. B. Maniatis, Molecular Cloning (1982) 280 - 281).

Vor Durchführung der Bestimmung können die Nucleinsäuren der sample in kürzerkettige Fragmente gespalten werden. Dies kann beispielsweise durch Ultraschallbehandlung, Mikrowellenbehandlung oder Behandlung mit Restriktionsendonucleasen geschehen. Falls die samples doppelsträngige Nucleinsäuren enthalten, müssen diese vor Durchführung der Bestimmung in Einzelstränge gespalten werden. Dies kann nach den dem Fachmann geläufigen Methoden durch Denaturierung (z.B. Hitzebehandlung oder alkalische Behandlung) erfolgen.

Die derivatisierte oder radioaktiv markierte Nucleinsäureprobe wird mit einer auf einen Träger gebundenen denaturierten DNA oder RNA der zu untersuchenden sample in Kontakt gebracht und hierbei Temperatur, Ionenstärke, pH-Wert, Puffer und sonstige Bedingungen, abhängig von der Länge der Nucleinsäureprobe und der daraus resultierenden Schmelztemperatur des zu erwartenden Hybrids so gewählt, daß die markierte Nucleinsäure an homologe Nucleinsäuren binden kann (J. Mol. Biol. 98 (1975), 503, Proc. Natl. Acad. Sci. USA 76 (1979) 368 3). Besonders geeignete Hybridisierungsbedingungen sind 5-6 x SSC bei 42 °C - 60 °C und eine Inkubationsdauer von 1 bis 20 Stunden, vorzugsweise 1 Stunde. Besonders geeignete Waschbedingungen sind 0,1 - 0,5 x SSC bei 65 °C - 70 °C und eine Inkubationsdauer von 15 Minuten bis zwei Stunden, vorzugsweise 30 Minuten. Zur Durchführung der Bestimmung ist es jedoch auch möglich, andere Bedingungen zu wählen, da die optimalen Bedingungen von der Art und Konzentration der probe und der zu bestimmenden Nucleinsäure abhängig sind. So kann beispielsweise durch Zusatz von 40 % Formamid eine niedrigere Hybridisierungstemperatur verwendet werden. Ebenso kann ein organisches Lösungsmittel in einer Konzentration bis zu 50%, vorzugsweise 20-50%, zugesetzt werden. Geeignete Hybridisierungsbedingungen lassen sich, wie in Anal. Biochem. 178(1984) 267, beschrieben, aus Länge der probe, Salzgehalt des Reagenzes, GC-Gehalt der probe und Temperatur berechnen. Als besonders geeignete Bedingungen haben sich erwiesen:

GC-Gehalt der probe 43% - 60%,
Länge der probe mindestens 27 Nucleotide,
Hybridisierung bei 42 °C - 60 °C und
bei 5 - 6 x SSC über 1 Stunde,
waschen bei 65 °C - 70 °C und
bei 0,1 - 0,5 x SSC über 30 Minuten,
ggf. unter Zusatz von
40% bis 50% Denaturierungsmittel (z. B. Formamid) während der Hybridisierung
Unter 1 x SSC ist eine wäßrige Lösung zu verstehen aus
0,15 mol/l NaCl
0,015 mol/l $Na_3$ Citrat x 2 $H_2O$,
welche mit 1 mol/l HCl auf pH 7 eingestellt ist. Angaben wie 0,1 x SSC sind entsprechende Verdünnung dieser Lösung.

Als Träger geeignet sind Membranen oder Trägermaterialen auf Basis Nitrocellulose (z.B. Schleicher und Schüll BA85, Amersham Hybond C), verstärkte oder gebundene pulverförmige Nitrocellulose oder mit verschiedenen funktionellen Gruppen (z.B. Nitrogruppe) derivatisierte Nylonmembranen (z.B. Schleicher und Schüll Nytran, NEN Gene Screen, Amersham Hybond N, Pall Biodyne). Vor Durchführung der Bestimmung wird der Träger vorzugsweise vorbehandelt, um nichtspezifische Bindungen der probes an den Träger zu verhindern. Für diese Vorhybridisierung geeignete Blockierungssubstanzen sind beispielsweise phosphate buffered saline mit Rinderserumalbumin, nichtionische Detergentien, Polyanionen und DNA aus Heringssperma. Nitrozellulosefilter und Nylonmembranen werden vorzugsweise mit 5 x SSC bei 65°C 1 Stunde vorbehandelt.

Nach Durchführung der Inkubation wird der Träger gewaschen, um unhybridisierte Nucleinsäuren zu entfernen. Dies kann beispielsweise erfolgen durch eine Lösung von 0,1 - 1 % (v/v) eines ionischen Detergenz, ggf. unter Zusatz von Salz (z. B. Natriumchlorid oder Natriumcitrat) oder mit SSC, vorzugsweise 0,1 - 0,5 x SSC.

Bei Verwendung von radioaktiv markierten probes wird eine Autoradiographie durchgeführt.

Bei Verwendung von Hapten-markierten probes wird mit einem Antikörper oder Antikörperfragment gegen das Hapten inkubiert. Der Antikörper trägt hierbei eine Markierung, beispielsweise eine radioaktive oder enzymatische Markierung. Nach der Antikörper-Inkubation wird nochmals gewaschen, um nur spezifisch gebundene Antikörper-Konjugate nachzuweisen. Die Bestimmung erfolgt dann über die Markierung des Antikörpers oder des Antikörperfragments nach den an sich bekannten Methoden. Entsprechendes gilt für eine Biotin- oder Avidinmarkierung.

Die Markierung des Anti-Hapten-Antikörpers oder des Antikörper-Fragments erfolgt in an sich bekannter Weise. Geeignet sind z. B. Enzymmarkierung, radioaktive Markierung, (Bio)Lumineszenz-Markierung oder Fluoreszenzmarkierung. Bevorzugt wird jedoch eine Enzymmarkierung mit Enzymen, wie alkalische Phosphatase, Peroxidase oder $\beta$-Glactosidase verwendet. Besonders bevorzugt wird als Markierungsenzym alkalische Phosphatase verwendet. Die Bestimmung der alkalischen Phosphatase erfolgt über Leukosysteme, insbesondere über indigoide Systeme als oxidierbare Verbindungen (vgl. EP-A 0228663). Als Oxidationsmittel dienen Tetrazoliumsalze. Bei dem Markierungsenzym alkalische Phosphatase wird als Redoxsystem bevorzugt X-Phosphat/Nitroblau-Tetrazolium eingesetzt (F.P. Altmann, Tetrazoliumsalts and Formazans, Progr. Histochem. Cytochem. vol. 913 (1976), Gustav-Fischer-Verlag Stuttgart, Seite.1). Unter X-Phosphat ist hierbei 5-Brom-4-chlor-3-indolylphosphat und unter Nitroblau-Tetrazolium 3,3'-(3,3'-Dimethoxy-4,4'-biphenylen)-bis-5-phenyl-2-(4-nitrophenyl)-tetrazoliumchlorid zu verstehen. Alkalische Phosphatase spaltet das chromogene Substrat (X-Phosphat), das durch die Abspaltung des Phosphats und Oxidation ein blaues, schwerlösliches Dimäres bildet, welches gleichzeitig die Tetrazoliumverbindung zu einem ebenfalls blauen, schwerlöslichen Formazan reduziert wird.

Der Nachweis der anderen geeigneten Markierungssysteme wird nach an sich bekannten Methoden durchgeführt.

Ein weiterer Gegenstand der Erfindung ist ein Reagenz zur Bestimmung von Listeriosis, welches dadurch gekennzeichnet ist, daß es eine markierte Nucleinsäure, die mindestens eine Länge von 27 Nucleotiden hat und bei 5 - 6 x SSC und 42°C - 60°C mit dem 2,5kb großen KpnI-BamHI Fragment aus dem Plasmid pLM63 (DSM 5220) hybridisiert. Bevorzugt ist ein Reagenz, welches eine markierte Nucleinsäure enthält, welche mit einer Nucleinsäure gemäß Tabelle III unter den oben genannten Bedingungen hybridisiert. Weiter bevorzugt ist ein Reagenz, welches eine markierte Nucleinsäure enthält, welche mit einer Nucleinsäure entsprechend den Nucleotiden 822 - 1890 aus Tabelle III unter den oben genannten Bedingungen hybridisiert. Vorzugsweise beträgt die Hybridisierungsdauer 1 - 20 Stunden, besonders bevorzugt 1 Stunde. Besonders geeignet ist, bei 0.1 - 0.5 x SSC und 65°C - 75°C zu waschen, wobei eine Inkubationsdauer von 15 Minuten bis zwei Stunden bevorzugt ist. Als ganz besonders bevorzugt hat sich eine Inkubationsdauer von 30 Minuten erwiesen. Außerdem enthält das Reagenz ein Detektionssystem für die Markierung. Zur Markierung können die oben beschriebenen Verfahren verwendet werden.

Vorzugsweise wird eine markierte Nucleinsäure entsprechend der Sequenz von Tabelle III oder entsprechend den Nucleotiden 1 - 714, 822 - 1890, 935 bis 1244, 1158 bis 1185 oder 1209 bis 1718 verwendet.

Ein weiterer Gegenstand der Erfindung ist ein Protein, welches zu mindestens 80 % homolog zu der in Tabelle III angegebenen Aminosäuresequenz Protein I, Protein II oder Protein III ist. Bevorzugt ist ein Protein, welches zu mindestens 80 % homolog zu Protein II ist. Dieses Protein hat ein Molekulargewicht von 18 kD. Die Proteine sind dazu geeignet, Antikörper gegen Listeria herzustellen. Bevorzugt sind die Proteine mit der Aminosäuresequenz gemäß Tabelle III oder den Teilsequenzen Protein I, Protein II oder Protein III. Besonders bevorzugt ist Protein II.

4

Mit dem gegebenenfalls modifizierten erfindungsgemäßen Protein lassen sich durch Immunisierung von Versuchstieren, Gewinnung von Antiserum und Reinigung der Antikörper nach ansich bekannten Verfahren Antikörper und Antiseren gegen pathogene Listeria-Bakterien erhalten. Monoklonale Antikörper können erhalten werden durch Immunisierung von Versuchstieren mit gegebenenfalls modifiziertem erfindungsgemäßen Protein, Fusion von B-Lymphozyten der so erhaltenen immunisierten Tiere mit transformierenden Agentien, Klonierung und Kultivierung der so gebildeten Hybridzellen, welche den monoklonalen Antikörper produzieren und Isolierung des letzteren. Besonders geeignete Tiere für die Herstellung der Antikörper sind Ratten und Mäuse. Geeignete Modifizierungsmittel sind beispielsweise N-Bromsuccinimid (NBS) unter Oxidation von Tryptophangruppen am Protein (BBA 143 (1967) 462-472) Carboximethylierung mit Jodacetat (IAA) die hauptsächlich am Histidin angreift bzw. Nitrierung mit Tetranitromethan (TNM) (J.Biol.Chem. 238 (1963) 3307) sowie die Azotierung mit diazotierter Sulfanylsäure (Meth.Enzymol. 25 (1972) 515-531). Besonders geeignete Versuchstiere sind Balb/c-Mäuse oder AJ-Mäuse.

Die Immunisierung erfolgt durch übliche Verabreichung des nativen oder modifizierten Enzyms, vorzugsweise in Kombination mit Adjuvans. Bevorzugt wird als Adjuvans Freund'sches Adjuvans oder Aluminiumhydroxid zusammen mit Bordetella pertussis verwendet. Die Immunisierung erfolgt üblicherweise über mindestens 2, vorzugsweise 4 Monate.

Zur Gewinnung monoklonaler Antikörper werden nach erfolgter Immunisierung die B-Lymphozyten der immunisierten Tiere nach üblichen Methoden mit transformierenden Agentien fusioniert. Beispiele für transformierende Agentien, die im Rahmen der Erfindung verwendet werden, sind Myelomazellen,transformierende Viren, wie z. B. Epstein-Barr Virus oder die in der DE-A 32 45 665 beschriebenen Agentien. Die Fusionierung erfolgt nach dem bekannten Verfahren von Köhler und Milstein (Nature 256 (1975) 495-497). Die hierbei gebildeten Hybridzellen werden in üblicher Weise kloniert, z. B. unter Verwendung eines handelsüblichen Zellsorters und die erhaltenen Klone, welche den gewünschten monoklonalen Antikörper bilden, gezüchtet. Die Herstellung und Selektion von monoklonalen Antikörpern ist beispielsweise in J.Immunol.Meth., 39 (1980) 285-308 ausführlich beschrieben.

Ein weiterer Gegenstand der Erfindung ist ein immunologisches Verfahren, zur Bestimmung von Listeria-Bakterien, bei dem mindestens ein ggf. monoklonaler oder polyklonaler Antikörper gegen das erfindungsgemäße Protein verwendet wird. Als immunologische Bestimmungsmethoden eignen sich im Prinzip alle gängigen Immuno-assays, wie z.B.Radio-Immunoassay, Enzym-Immunoassay, Floureszenz-Immunoassay u.s.w.. Ferner sind sämtliche Verfahrensvarianten, wie z.B. kompetitiver Immuno-assay JEMA-Verfahren anwendbar. Zur Markierung eignen sich die für die jeweilige Bestimmungsmethode üblichen Mittel. So werden bei einem Radio-Immunoassay Radio-Isotope, beispielsweise $^{125}$J, zur Markierung verwendet. Für einen Enzym-Immunoassay sind sämtliche hierfür üblicherweise eingesetzten Enzyme, beispielsweise Peroxidase oder $\beta$-Galactosidase, geeignet. Für eine Floureszenz-Immunoassay sind die üblichen floureszierenden Gruppen als Markierung brauchbar. Einzelheiten dieser verschiedenen Testmethoden und Verfahrensvarianten sind dem Fachmann bekannt.

In dem Bestimmungsverfahren können die monoklonalen oder polyklonalen Antikörper als solche oder Fragmente hiervon, welche die entsprechenden immunologischen Eigenschaften aufweisen, beispielsweise Fab-Fragmente, verwendet werden.

Die folgenden Beispiele und die Abbildungen erläutern die Erfindung weiter. Sofern nichts anderes vermerkt, sind alle Prozentangaben Gewichtsprozentangaben. Tabelle III zeigt die DNA-Sequenzen von dth 18 (Nucleotid 822 - 1890) und eines bevorzugten Ausschnittes aus dem 2,5 kb großen KpnI-BamHI Fragment von Plasmid pLM63 (Nucleotid 1 - 1890) sowie die Aminosäuresequenzen der Proteine I, II und III einschließlich Startcodon.

Beispiel 1

Markierung der DNA-probes

200 ng der isolierten DNA werden $^{32}$P-markiert, wie in Anal. Biochem. 132 (1983), 6-13 beschrieben.

Beispiel 2

Probenvorbereitung

2.1 Mikroorganismen

Die verwendeten Referenzstämme sind in Tab. Ia aufgelistet. Alle anderen Listeria-Stämme (Tabellen Ib, II) wurden aus einer Sammlung von etwa 3000 Listeria-Stämmen des National Institute of Public Health and Environmental Protection, Bildhoven, ausgewählt. Unter den geprüften Stämmen befinden sich 40 Stämme, die von an Listeriose erkrankten Patienten stammen. Diese Stämme wurden, wie in Zbl. Bact. I Abt. Orig. A 246 (1980), 211 - 227 beschrieben, isoliert. Die Mikroorganismen wurden in einem flüssigen Selektions- und Anreicherungsmedium (pH 7,0 1g/l Pepton, 8,5g/l NaCl, 10$\mu$g/ml, Trypaflavin•HCl, 10$\mu$g/ml Nalidixinsäure, 50$\mu$g/ml Cyclohexamid) über 24 Stunden bei 30°C gezüchtet und anschließend auf Spezialnährboden (Anreicherungsmedium + 1% Agar) ausplattiert. Nach Bebrütung bei 30°C über 24 Stunden werden diese Platten direkt in dem Hybridisierungstest eingesetzt.

2.2 Anderes Probenmaterial

Als weiteres Probenmaterial wird eingesetzt Cerobrospinalflüssigkeit, faeces, Blut, Lochia, Gehirnmaterial, Leber, Cervixmaterial, Amnionfluid, Lebensmittel, Silagegras und Tiermaterial. Von diesem Material werden 20 g in 250 ml phosphate buffered saline suspendiert, homogenisiert und weiter aufgearbeitet, wie in International Journal of Food Microbiology 4 (1987), 249 - 256 beschrieben. 0,1 ml der Probe werden auf nichtselektive Agarplatten (85 mm Durchmesser) ausplattiert und über Nacht inkubiert.

2.3 Durchführung eines Hybridisierungstests mit einer DNA-probe (dth 18, 1069 Nucleotide oder pLM63, 1890 Nucleotide, Tabelle III)

Von den nach 2.1 bzw. 2.2 hergestellten Platten wird mit Nylonmembranen (Gene Screen plus membranes[R], DuPont Corp., USP 4,455,370) ein Abklatsch hergestellt. Die Membranen werden auf Filterpapier gelegt, welches mit 0,5 mol/l NaOH getränkt ist und für 5 Minuten im kochenden Wasserbad inkubiert. Anschließend werden die Membranen auf Filterpapier gebracht, welches mit 1 ml frischer 0,5 mol/l NaOH getränkt ist und mit 1 ml 1 mol/l Trispuffer (pH 7,5) neutralisiert. Dieser Neutralisationsschritt wird wiederholt. Die Membranen werden in 100 ml 5 x SSC getaucht und dabei mit einem Tuch die Zellreste abgerieben. Nach Lufttrocknung werden die Membranen über 6 - 16 Stunden bei 40°C mit 15 ml Lösung A vorhybridisiert. Anschließend wird die DNA-probe dth 18 oder pLM63 (0,05 $\mu$g in 2,5 ml 5 x SSC) zugegeben bei 60°C für 18 Stunden inkubiert. Anschließend wird 45 min mit 0,2 x SSC, welches zusätzlich 1% SDS und 0,1 % Natriumpyrophosphat enthält, im Wasserbad bei 65°C gewaschen. Nach Trocknung werden die Membranen in Plastikfolie eingeschweißt und damit bei -70°C über 18 Stunden ein Röntgenfilm belichtet.

2.4 Hybridisierung mit einer 314bp DNA-probe

Hybridisierungstests werden mit einer verkürzten probe (Nucleotid 1209 - 1716 (vgl. Tabelle III) durchgeführt. Die Hybridisierungsbedingungen sind 5 x SSC bei 42°C (1 Stunde) und anschließendes Waschen in 0,2 x SSC bei 70°C (30 Minuten). zu sehen. Es zeigt sich, daß auch diese probe als DNA-probe zur Prüfung auf Listeriose geeignet ist.


Reagentien:


1 x SSC: 0,15 mol/l NaCl und 0,015 mol/l Natriumcitrat


Lösung A:


50 mmol/l Trispuffer, pH 7,5,
10 mmol/l EDTA
1 mol/l NaCl
0,2 % Ficoll
0,2 % Polyvinylpyrrolidon
0,2 % Rinderserumalbumin
1 mg/ml denaturierte Heringssperma-DNA
1 % Natriumpyrophosphat und
10 % SDS (Natriumdodecylsulfat).

Zur weiteren Überprüfung wurde noch mit weniger stringenten Bedingungen (2 x SSC bei 65°C) gearbeitet. Dabei ergaben sich analoge Ergebnisse.


Beispiel 3


Vergleichsversuche mit einer DNA-probe nach dem Stand der Technik (Listerolysin O)

Es wurde als probe ein synthetisches 19 mer-oligomer ([5]GATCACTCTGGAGGATACG[3]) verwendet (analog Infection and Immunity (56 (1988), 766 - 772)

200 ng dieser DNA-Probe wurde am 5'-Ende mit $^{32}$P-markiert, wie in Maxam und Gilbert (Meth. Enzymol. 65 (1980, 499) beschrieben.

Die Hybridisierungsbedingungen waren:

6 x SSC bei 37°C, dreimaliges Waschen mit 6 x SSC, enthaltend 1 % SDS und 0,1 % Natriumpyrophosphat für jeweils 30 min bei 40°C.

Beispiel 4

Biotypisierung der verwendeten Mikroorganismen

Die Biotypisierung wurde, wie in An. Inv. Pasteur/ Microbiol. 134 (1983) 56 - 71 beschrieben, durchgeführt. Die Stämme wurden in 4 ml halbfestem Peptonagarmedium, welches 1 % D-Xylose oder L-Ramnose enthält, zwei Tage bei 37°C angezogen. Das Peptonmedium besteht aus 10 g Bacto-Pepton, 5 g Natriumchlorid, 5,5 Oxoidagar L 28 und 0,08 g Bromthymolblau aufgelöst in 1000 ml destilliertem Wasser, pH 7,8, autoklaviert bei 120°C für 15 min. Nach Autoklavieren wird filtersterilisierte Zuckerlösung zugegeben bis zu einer Endkonzentration von 1 %. Zur Biotypisierung wurde die Hämolysinproduktion geprüft durch Züchtung der Stämme über Nacht bei 37°C in Brain Heart-Infusionsbrühe. Von dieser Kultur werden 0,2 ml entnommen und mit 0,2 ml einer 2 %igen Schaferythrozytensuspension in PBS (Phosphat buffered saline), welche dreimal gewaschen wurde, zugegeben. Nach zweistündiger Inkubation bei Raumtemperatur wird auf Hämolyse geprüft. L. monocytogenes, NCTC7973 und L. innocua, NCTC11289 dienten als positive und negative Kontrollen.

Die Biotypisierung erfolgt nach Zbl. Bakt. Mikrobiol. Hyg. I Abt. Orig. A 259 (1985) 341 - 350.

Beispiel 5

Serotypisierung

Die Serotypisierung wurde, wie in Seeliger und Höhne (Zbl. Bact. Microbiol. Hyg. I Abt. Orig. A, 259 (1985), 341-350 beschrieben, durchgeführt.

Die Stämme wurden in 4 ml Tryptonphosphat-Brühe, welche mit 1 % Glucose angereichert ist, angezüchtet und im Wasserbad bei 37°C für 6 - 7 Stunden geschüttelt. Mit dieser Kultur wird eine Tryptoseagarplatte inokuliert und über Nacht bei 37°C inkubiert. Parallel wird ein zweites Röhrchen mit Tryptosephosphat-Brühe, welcher 1 % Glucose zugegeben wird, inokuliert und bei 25°C unter Schütteln über Nacht im Wasserbad inkubiert. Die Tryptosephosphat-Platte wird mit 5 ml phosphate buffered saline, pH 7,4 geerntet, eine Stunde im kochenden Wasserbad erhitzt und anschließend zentrifugiert.

Die Zellen werden resuspendiert und auf eine Konzentration von $5 \times 10^8$ Zellen/ml eingestellt ($1 \times 10^9$ Zellen/ml haben eine OD von 1,0 bei 600 nm). Von dieser Suspension werden 0,2 ml entnommen und zu 0,2 ml 75fach verdünntem Antiserum gegen die Polysaccharid-Antigene I, II, V, VI, VIII und IX im Glasröhrchen gegeben und bei 45°C für zwei Stunden inkubiert. Anschließend wird abgekühlt und über Nacht bei 4°C inkubiert. Anschließend werden die Röhrchen auf sichtbare Agglutination geprüft.

Die Serotypisierung erfolgt, wie in Bergeys Manual of Bacteriology (N.R.Kreig, J.G.Holt (eds) Bergeys Manual of Systematic Bacteriology (1984),Williams and Wilkins, Baltimore and London) näher beschrieben. Es wurde in die Serotypenklassen 1/2a, 1/2b, 1/2c, 3a, 3b, 3c, 4a, 4b, 4d, 4a/b, 5, 6b und 7 eingeteilt.

Beispiel 6

Maus-Bioassay

Zur Überprüfung, ob ein Listeriosis-Stamm pathogen ist, wird ein Maus-Bioassay durchgeführt, wie in Infect. Immun. 45 (1984), 234 - 241 beschrieben, durchgeführt. Dazu werden $10^4$ - $10^5$ Bakterien in 200 $\mu$l phosphate buffered saline pH 7,4 intravenös in die Maus injiziert. Nach 2 - 4 Tagen werden die Mäuse getötet und die Milz entnommen. Als pathogen wird ein Stamm dann bezeichnet, wenn die Anzahl der Bakterien, die in der gesamten Milz gefunden werden, $10^4$ übersteigt.

Beispiel 7

Herstellung von Listeria Antikörpern

50 $\mu$g des gereinigten Antigens (Aminosäuresequenz Tabelle III) werden in complete Freund'schem Adjuvans intramuskulär (IM) injiziert. Drei Wochen, 30 und 40 Tage später wird die Injektion wiederholt. 10 Tage nach der letzten Injektion wird Antiserum gewonnen und affinitätschromatographisch gereinigt.

Mit diesem Antiserum kann ein Immunoblot zur Bestimmung von Listeria durchgeführt werden.

Beispiel 8

Überprüfung der Eignung einer probe für eine Listeria-Bestimmung

Mit einem Plasmid auf Basis von pUC18, welches die DNA-Sequenz nach Tabelle III insertiert enthält, werden E.coli HB 101 (DSM 1607) transformiert und in Gegenwart von 50 $\mu$g/ml Ampicillin, bis zu einer optischen Dichte von OD 600nm = 1 über Nacht gezüchtet. 0,1 ml dieser Kulturbrühe werden aus Agarplatten, die 50$\mu$g/ml Ampicillin enthalten (85 mm Durchmesser) ausplattiert und über Nacht inkubiert.

Von den so hergestellten Platten wird mit Nylonmembranen (Gene Screen Plus-Membranes[R], DuPont) ein Abklatsch hergestellt.

Die Membranen werden auf Filterpapier gelegt, welches mit 0,5 mol/l NaOH getränkt ist und für 5 Minuten im kochenden Wasserbad inkubiert. Anschließend werden die Membranen auf Filterpapier gebracht, welches mit 1 ml frischer 0,5 mol/l NaOH getränkt ist und mit 1 ml 1 mol/l Trispuffer, pH 7,5, neutralisiert. Dieser Neutralisationsschritt wird wiederholt. Die Membranen werden in 100 ml 5 x SSC getaucht und dabei mit einem Tuch die Zellreste abgerieben. Nach Lufttrocknung werden die Membranen über 6 - 16 Stunden bei 40°C mit 15 ml Lösung A vorhybridisiert. Anschließend wird die auf Eignung zu überprüfende DNA-probe (0,05 $\mu$g in 2,5 ml) zugegeben und bei 60°C für 18 Stunden inkubiert. Anschließend wird 45 Minuten in 0,2 x SSC, welches zusätzlich 1 % SDS und 0,1 % Natriumpyrophosphat enthält, im Wasserbad bei 65°C gewaschen. Nach Trocknung werden die Membranen in Plastikfolien eingeschweißt und damit bei minus 70°C über 18 Stunden ein Röntgenfilm belichtet. Wenn auf dem Röntgenfilm Signale ersichtlich sind, die von einer Hybridisierung stammen, ist diese probe zur Verwendung im Listeriosistest geeignet. Analoge Ergebnisse werden erhalten, wenn als Plasmid pLM63 (DSM 5220) verwendet wird.

Beispiel 9

Vergleich der Hybridisierung einer erfindungsgemäßen probe und einer Vergleichsprobe (vgl. Beispiel 3) mit verschiedenen Listeriosis-Stämmen. Die Hybridisierung wurde wie in Beispiel 2 und 3 beschrieben durchgeführt.

Die Ergebnisse zeigen Tabelle I und II.

**Tabelle I**

Vergleich der Hybridisierung einer erfindungsgemäßen DNA-probe (1) (935bp bis 1244bp von Tabelle III) und einer DNA-probe nach dem Stand der Technik (Listeriolysin O,(2), hergestellt nach Beispiel 3) mit DNA aus Listeria-Stämmen.

Analoge Ergebnisse werden mit Nucleotid 1 - 714 als DNA-Probe (1) erzielt. Zusätzlich hybridisiert diese Probe noch mit DNA aus Listeria monocytogenes Stämmen des Serotyps 4a (z.B. NCTC 5214).

**Tabelle Ia:**
mit Referenzstämmen

| Sero-typ | Stamm-Nr.[1] | Biotyp | probe 1 | 2 |
|---|---|---|---|---|
| 1/2 a | NCTC7973 | L.monocytogenes | + | + |
| 1/2 b | SLCC2755 | L.monocytogenes | + | + |
|  | SLCC3954 | L.seeligeri | - | - |
| 1/2 c | NCTC5348 | L.monocytogenes | + | + |

| Sero-typ | Stamm-Nr. | Biotyp | probe 1 | 2 |
|---|---|---|---|---|
| 3 a | NCTC5105 | L.monocytogenes | + | + |
| 3 b | SLCC2540 | L.monocytogenes | + | + |
| 3 c | SLCC2479 | L.monocytogenes | + | + |
| 4 b | NCTC10527 | L.monocytogenes | + | + |
| 4 c | ATCC19116 | L.monocytogenes | + | + |
| 4 d | NCTC10888 | L.monocytogenes | + | + |
| 4 e | ATCC19118 | L.monocytogenes | + | + |
| 4 ab | NCTC10528 | L.monocytogenes | + | + |
| 5 | ATCC19119 | L.ivanovii | + | - |
| 6 a | SLCC 5334 | L.welshimeri | - | - |
|  | NCTC11288 | L.innocua | - | - |
| 6 b | NCTC11289 | L.innocua | - | - |
| 7 | SLCC 2482 | L.monocytogenes | + | + |
|  | RIVM 1 | L.grayi | - | + |
|  | RIVM 2 | L.murravi | - | + |

[1] Hinterlegungstellen vgl. World Directory of Collections of Cultures of Microorganisms edited by V.F. McGowan and V.B.D. Sherman, World Data Center, University of Queensland, Australien, 1982.

**Tabelle I b:**
mit einer Vielzahl von Stämmen

| Anzahl der untersuch- ten Stämme | Biotyp | | probe | |
|---|---|---|---|---|
| | | | 1 | 2 |
| 34 | L.monocytogenes | (34) | 34 | 34 |
| 39 | L.monocytogenes | (37) | 37 | 37 |
| | L.seeligeri | ( 1) | 0 | 0 |
| | L.welshimeri | ( 1) | 0 | 0 |
| 16 | L.monocytogenes | (16) | 16 | 16 |
| 5 | L.monocytogenes | ( 5) | 5 | 5 |
| 5 | L.monocytogenes | ( 3) | 3 | 3 |
| | L.welshimeri | ( 1) | 0 | 0 |
| | L.seeligeri | ( 1) | 0 | 0 |
| 2 | L.monocytogenes | ( 1) | 1 | 1 |
| | L.innocua | ( 1) | 0 | 0 |
| 77 | L.monocytogenes | (77) | 76 | 76 |

| Anzahl der untersuch-ten Stämme | Biotyp | | probe | |
|---|---|---|---|---|
| | | | 1 | 2 |
| 5 | L.seeligeri | ( 5) | 0 | 0 |
| 2 | L.monocytogenes | ( 2) | 2 | 2 |
| 1 | L.mcnocytogenes | ( 1) | 1 | 1 |
| 34 | L.innocua | (32) | 0 | 1 |
| | L.welshimeri | ( 2) | 0 | 0 |
| 23 | L.innocua | (18) | 0 | 2 |
| | L.welshimeri | ( 5) | 0 | 0 |
| 7 | L.monocytogenes | ( 7) | 7 | 7 |
| 2 | L.grayi | ( 2) | 0 | 2 |
| 3 | L.murrayi | ( 3) | 0 | 3 |

## Tabelle II

Tabelle II zeigt die Hybridisierungsreaktion von probe 1 mit
verschiedenen Listeria-Stämmen, deren Pathogenität
nach Beispiel 6 bestimmt wurde.

| Biotyp | Sero-typ | Herkunft | Hybridi-sierung | log Anzahl/g Milz | |
|---|---|---|---|---|---|
| | | | | 2. Tag | 4. Tag |
| L.monocytogenes | 1/2 a | Referenz-stamm | + | 7.4[c] | 8.3 + |
| L.monocytogenes | 1/2 b | Weich-käse | + | 6.2 | 7.1 + |
| L.monocytogenes | 1/2 c | Lebens-mittel | + | 5.4 | + + |
| L.monocytogenes | 3a | Lebens-mittel | + | 6.4 | 6.5 |
| L.monocytogenes | 3 b | Lebens-mittel | + | 8.4 | + + |
| L.monocytogenes | 3 c | Lebens-mittel | + | 8.2 | + + |
| L.monocytogenes | 4 b | Weich-käse | + | 7.0 | + + |
| L.monocytogenes | 4 b | Weich-käse | + | 6.5 | + + |
| L.monocytogenes | 4 b | Weich-käse | + | 8.3 | + + |
| L.seeligeri | 4 c | Lebens-mittel | - | <2.0 | <2.0· |

13

| Biotyp | Serotyp | Herkunft | Hybridisierung | log Anzahl/g Milz | |
|--------|---------|----------|----------------|-------------------|---|
| | | | | 2. Tag | 4. Tag |
| L.innocua | 6 a | Weichkäse | - | 2.0 | 3.7 |
| L.innocua | 6 b | Weichkäse | - | <2.0 | <2.0 |
| L.grayi | | Referenzstamm | - | <2.0 | <2.0 |
| L.murrayi | | Referenzstamm | - | <2.0 | <2.0 |

a) $10^4$-$10^5$ Organismen wurden 4 Mäusen injiziert.

b) Hybridisierung wird mit dem dth 18-gene als probe durchgeführt (vgl. Beispiel 2.3).c) Mittelwert von 2 Mäusen; +: Maus vor Probenahme gestorben.

Tabelle III

NUCLEOTIDE SEQUENCE OF PLASMID pPLM63

```
                10        20        30        40        50        60
                 |         |         |         |         |         |
     CTTTTCATTTAGATAAAACAAAAGAAGAAATTGGCGCTTTACCTGCTTCGGCGATTGAAT
      PheHisLeuAspLysThrLysGluGluIleGlyAlaLeuProAlaSerAlaIleGluCys


                70        80        90       100       110       120
                 |         |         |         |         |         |
     GTCAGTATGAGGCTTTTGTGATTAATGAAGCCAATAATTAAGGAGTGATAAAATGCAGGT
      GlnTyrGluAlaPheValIleAsnGluAlaAsnAsn---            METGlnVal
                                                      ↳ Protein III

               130       140       150       160       170       180
                 |         |         |         |         |         |
     TTTAGTTTTACCAGAAAATAAGGATATCAATTATATAAAAACGGTCCAAGAAGTAAAACG
      LeuValLeuProGluAsnLysAspIleAsnTyrIleLysThrValGlnGluValLysArg


               190       200       210       220       230       240
                 |         |         |         |         |         |
     ATTTTTTGCGGATTTTGAGCGGTTTCGGATGATTACGGGGGTTATCAAAAAAGCCACATTT
      PhePheAlaAspPheGluArgPheArgMETIleThrGlyLeuSerLysLysProHisLeu


               250       260       270       280       290       300
                 |         |         |         |         |         |
     ACTTAGAAATGGTTTTCTGGAAGAGCCGCAGTTTGAGCCGGTAGCATTTTCTGCTAGACA
      LeuArgAsnGlyPheLeuGluGluProGlnPheGluProValAlaPheSerAlaArgHis


               310       320       330       340       350       360
                 |         |         |         |         |         |
     TAATAAAGAAGTCATTTTGGAAGCGCGATGGTTGGTAGAGAAATATACTGAAATGTTGAA
      AsnLysGluValIleLeuGluAlaArgTrpLeuValGluLysTyrThrGluMETLeuAsn


               370       380       390       400       410       420
                 |         |         |         |         |         |
     TCAGATGGATGATTTATATCGAACTATTTTGATGGAATGTTACGTGGAACGAAAACAAGA
      GlnMETAspAspLeuTyrArgThrIleLeuMETGluCysTyrValGluArgLysGlnAsp


               430       440       450       460       470       480
                 |         |         |         |         |         |
     TGTGGCGGTAATGATGGATTTACCGTATGAAATTGCCCAGTTTAAACGGATAAAAAAACG
      ValAlaValMETMETAspLeuProTyrGluIleAlaGlnPheLysArgIleLysLysArg


               490       500       510       520       530       540
                 |         |         |         |         |         |
     GGCAGTGCTAGAACTTGCAACGCTAATGGGGATTTTAGTAAGGAAATGATGATACTTTCG
      AlaValLeuGluLeuAlaThrLeuMETGlyIleLeuValArgLys---
                                               ─────→ Protein III
```

Tabelle III (Forts.)

```
            550         560         570         580         590         600
             |           |           |           |           |           |
TGATATTTTGAAACATCATTTTCCTATTAATATAGAAGTAAGCTAATTGTCCAGTAAGCG


            610         620         630         640         650         660
             |           |           |           |           |           |
GATGACAATAAAAGCTGCATCAGAATGAAGGTGCACCGATTTTCTGATAATACATGATGT


            670         680         690         700         710         720
             |           |           |           |           |           |
TTTACAAGGAATTTGTTTTTATGATTGGATTTAAATCCGTTGAGATAAACAAATATTCTA


            730         740         750         760         770         780
             |           |           |           |           |           |
TTTTGGAAAGTAAAGTTCGGAGGAATAAATTATTAAATGTGGTCTTGACCGAACTTTGCT


            790         800         810         820         830         840
             |           |           |           |           |           |
TTCTGTTTTAAAGGAGTGAACGTTTGGTGAAGAGTTTGAGCTTCATGAGAGTTTTGGAAG
                             ValLysSerLeuSerPheMETArgValLeuGluAla
                                        └───→ Protein I

            850         860         870         880         890         900
             |           |           |           |           |           |
CAGTGAGAACAATGCTCCAGGAAAAAGGCGGACTAGATATTTCTATTGTAATGCGTGACC
   ValArgThrMETLeuGlnGluLysGlyGlyLeuAspIleSerIleValMETArgAspGly

            910         920         930         940         950         960
             |           |           |           |           |           |
AAGTGGAAATGCCTACAACGATGATCGAGATGATTGATCAAGAGGAAGAAGAAAGCCAAA
   ValGluMETProThrThrMETIleGluMETIleAspGlnGluGluGluGluSerGlnThr

            970         980         990        1000        1010        1020
             |           |           |           |           |           |
CTGCCTGGAAAGAAAAATACCGTTTTGCAATCCATCATTATACAAATGAAACGGACTTAG
   AlaTrpLysGluLysTyrArgPheAlaIleHisHisTyrThrAsnGluThrAspLeuAla

           1030        1040        1050        1060        1070        1080
             |           |           |           |           |           |
CGGGAGTCGAAAAGATAGATACGCTTATCCAAACAGGATTCACTTTGCCTGAAGGATACA
   GlyValGluLysIleAspThrLeuIleGlnThrGlyPheThrLeuProGluGlyTyrLys

           1090        1100        1110        1120        1130        1140
             |           |           |           |           |           |
AATTAATCGCTGTTCGACATTACGGAAAACAAAATTTAGTCAAAGAAAATACGTTAATTC
   LeuIleAlaValArgHisTyrGlyLysGlnAsnLeuValLysGluAsnThrLeuIleHis
```

Tabelle III (Forts.)

```
        1150      1160      1170      1180      1190      1200
         |         |         |         |         |         |
ACGCAAAAACCAGTTTTGAAGTAAGTATTTGTCGTGAATTAAAAGTAAAAATTTAGGGGG
  AlaLysThrSerPheGluValSerIleCysArgGluLeuLysValLysIle---
                                     ———————▶ ProteinI

        1210      1220      1230      1240      1250      1260
         |         |         |         |         |         |
AAATATTAATGGCATTTGAAGAGAATTTATATTGTGATTATACACCGGGAGCTGCTAAAG
     METAlaPheGluGluAsnLeuTyrCysAspTyrThrProGlyAlaAlaLysAla
       └———————▶ Protein II

        1270      1280      1290      1300      1310      1320
         |         |         |         |         |         |
CGGTCGCGGGGAAAGATGTAATTTTAGCAGTTTTTAACGCAGCGGGGGACAAACTATTAG
  ValAlaGlyLysAspValIleLeuAlaValPheAsnAlaAlaGlyAspLysLeuLeuAla

        1330      1340      1350      1360      1370      1380
         |         |         |         |         |         |
CGGTTGCGGGCCAACAAGGTCTAACTGTAAACCGTTCTAAAGATAGCATTGAAATTACAT
  ValAlaGlyGlnGlnGlyLeuThrValAsnArgSerLysAspSerIleGluIleThrSer

        1390      1400      1410      1420      1430      1440
         |         |         |         |         |         |
CTAAAGATACAGTTGGCGGATGGAAATCCAAAATTGGCGGTATGAAAGAATGGTCAATTG
  LysAspThrValGlyGlyTrpLysSerLysIleGlyGlyMETLysGluTrpSerIleGlu

        1450      1460      1470      1480      1490      1500
         |         |         |         |         |         |
AAAATGACGGATTATATGTCGCTGATGCAGAGTCTCACAAAGAATTGGCGAAATATTTCG
  AsnAspGlyLeuTyrValAlaAspAlaGluSerHisLysGluLeuAlaLysTyrPheGlu

        1510      1520      1530      1540      1550      1560
         |         |         |         |         |         |
AAAGTGATAGCCCGGTTTGTGTGAAAATCATTAATCAAGCATCTAAAAAAGGTCTTTTCG
  SerAspSerProValCysValLysIleIleAsnGlnAlaSerLysLysGlyLeuPheGly

        1570      1580      1590      1600      1610      1620
         |         |         |         |         |         |
GTGGTTTGGCAATTGTAGCTGACTATAGTTTTGAAGCACCTTTTGACGAAGCGATGACTT
  GlyLeuAlaIleValAlaAspTyrSerPheGluAlaProPheAspGluAlaMETThrTyr

        1630      1640      1650      1660      1670      1680
         |         |         |         |         |         |
ACTCTGTAAAACTAGACGGAATGGGCGCGCTTGTTGATTTAACGATTACTGAGGGCGGCG
  SerValLysLeuAspGlyMETGlyAlaLeuValAspLeuThrIleThrGluGlyGlyAsp

        1690      1700      1710      1720      1730      1740
         |         |         |         |         |         |
ACCAAATGCCCGGCGAAACACCTGTAGCACCAGCAGAATAAAATAGAAAGCCACTGAAAT
  GlnMETProGlyGluThrProValAlaProAlaGlu---
                        ———————▶ Protein II
```

17

Tabelle III (Forts.)

```
          1750         1760         1770         1780         1790         1800
           |            |            |            |            |            |
AAGTGGCTTTCCCTTAGGAGGAAAATAAATGTTTGAAGTGAATGATACAACTTATATTTT
                                        METPheGluValAsnAspThrThrTyrIleLeu


          1810         1820         1830         1840         1850         1860
           |            |            |            |            |            |
ACGATTTAATAAACAAAAAGTTAAAACGGTGGAATTAACATCAGGGATTAGTTTAGTTGC
ArgPheAsnLysGlnLysValLysThrValGluLeuThrSerGlyIleSerLeuValAla


          1870         1880         1890
           |            |            |
AGCTTTGACTGCGAATAAAGGGATTTTGAG
AlaLeuThrAlaAsnLysGlyIleLeu
```

**Patentansprüche**

1.  Nucleinsäure, welche bei den Hybridisierungsbedingungen 5 - 6 x SSC und 42 - 60°C und den Waschbedingungen 0.1 - 0.5 x SSC bei 65° bis 75°C mit dem 2,5 kb großen KpnI-BamHI-Fragment aus dem Plasmid pLM63 (DSM 5220) hybridisiert.

2.  Nucleinsäure nach Anspruch 1, dadurch gekennzeichnet, daß sie mit dem Nucleinsäurefragment nach Tabelle III hybridisiert.

3.  Nucleinsäure nach Anspruch 1 oder 2, dadurch gekennzeichnet, daß der GC-Gehalt der Nucleinsäure 43% bis 60% beträgt.

4.  Nucleinsäure nach Anspruch 1 bis 3, welche mit einem mindestens 27 bp langen Ausschnitt oder der gesamten Nucleinsäure-Sequenz von Tabelle III identisch ist.

5.  Nucleinsäure nach Anspruch 1 bis 4, dadurch gekennzeichnet, daß sie eine Markierung trägt.

6.  Nucleinsäure nach Anspruch 5, dadurch gekennzeichnet, daß sie radioaktiv, mit Biotin, Avidin, Streptavidin, einem Fluoreszenzfarbstoff oder einem Hapten markiert ist.

7.  Verfahren zur Bestimmung von Listeria-Bakterien, dadurch gekennzeichnet, daß die zu untersuchende Probe mit einer Nucleinsäure, welche mit einem mindestens 27 bp langen Ausschnitt oder der gesamten Nucleinsäure-Sequenz von Tabelle III identisch ist und eine Markierung trägt, inkubiert wird und die Markierung durch ein geeignetes Nachweissystem bestimmt wird.

8.  Verfahren nach Anspruch 7, dadurch gekennzeichnet, daß die Hybridisierung bei einer Hybridisierungstemperatur von 42°C bis 60°C und 5 - 6 x SSC durchgeführt und anschließend bei 65° bis 75°C und 0.1 - 0.5 x SSC gewaschen wird.

9.  Protein, welches zu mindestens 80% homolog zu der Aminosäuresequenz I, II oder III von Tabelle III ist.

10. Protein nach Anspruch 9 mit der Aminosäuresequenz Protein I, Protein II oder Protein III.

11. Verwendung der Proteine nach den Ansprüchen 9 oder 10 zur Herstellung von Antikörpern gegen Listeria-Bakterien.

**12.** Antikörper erhältlich durch Immunisierung von Versuchstieren mit einem Protein nach den Ansprüchen 9 oder 10.

**13.** Verwendung von Antikörpern nach Anspruch 12 zur immunologischen Bestimmung von Listeria-Bakterien.

**Claims**

**1.** Nucleic acid which, in the case of the hybridisation conditions 5 - 6 SSC and 42 - 60°C and the wash conditions 0.1 - 0.5 x SSC at 65° to 75°C, hybridises with the 2.5 kb sized KpnI-BamHI fragment from the plasmid pLM63 (DSM 5220).

**2.** Nucleic acid according to claim 1, characterised in that it hybridises with the nucleic acid fragment according to Table III.

**3.** Nucleic acid according to claim 1 or 2, characterised in that the GC content of the nucleic acid amounts to 43 to 60%.

**4.** Nucleic acid according to claim 1 to 3, which is identical with an at least 27 bp long segment or the whole nucleic acid sequence of Table III.

**5.** Nucleic acid according to claim 1 to 4, characterised in that it carries a labelling.

**6.** Nucleic acid according to claim 5, characterised in that it is labelled radio-actively, with biotin, avidin, streptavidin, a fluorescent coloured material or a hapten.

**7.** Process for the determination of Listeria bacteria, characterised in that the sample to be investigated is incubated with a nucleic acid which is identical with an at least 27 bp long segment or the whole nucleic acid sequence of Table III and carries a labelling and the labelling is determined by a suitable detection system.

**8.** Process according to claim 7, characterised in that the hybridisation is carried out at a hybridisation temperature of 42°C to 60°C and 5 - 6 x SSC and is subsequently washed at 65° to 75°C and 0.1 - 0.5 x SEC.

**9.** Protein which is at least 80% homologous to the amino acid sequence I, II or III of Table III.

**10.** Protein according to claim 9, with the amino acid sequence protein I, protein II or protein III.

**11.** Use of proteins according to claims 9 or 10 for the production of antibodies against Listeria bacteria.

**12.** Antibodies obtainable by immunisation of experimental animals with a protein according to claims 9 or 10.

**13.** Use of antibodies according to claim 12 for the immunological determination of Listeria bacteria.

**Revendications**

**1.** Acide nucléique qui s'hybride avec le grand fragment KpnI-BamHI de 2,5 kb du plasmide pLM63 (DSM 5220) quand l'hybridation est réalisée dans du SSC 5-6 x, à 42°C-60°C et le lavage avec du SSC 0,1 - 0,5 X, entre 65°C et 75°C.

**2.** Acide nucléique selon la revendication 1, caractérisé en ce qu'il s'hybride avec le fragment d'acide nucléique comme indiqué dans le tableau III.

**3.** Acide nucléique selon la revendication 1 ou 2, caractérisé en ce que le contenu en GC de l'acide nucléique est compris entre 43 % et 60 %.

**4.** Acide nucléique selon les revendications 1 à 3 qui est identique à la séquence complète de l'acide nucléique du tableau III ou à la séquence d'un fragment de celle-ci ayant une longueur d'au moins 27 pb.

**5.** Acide nucléique selon les revendications 1 à 4, caractérisé en ce qu'il est marqué.

**6.** Acide nucléique selon la revendication 5, caractérisé en ce qu'il est radioactif ou marqué par de la biotine, de l'avidine, de la streptavidine, une substance fluorescente ou un haptène.

**7.** Procédé pour la détermination de bactéries du genre Listeria, caractérisé en ce qu'on laisse incuber l'échantillon à analyser avec un acide nucléique marqué et identique à une section longue d'au moins 27 pb ou à toute la séquence de l'acide nucléique du tableau III et en ce que le marquage est révélé par un procédé de détection approprié.

**8.** Procédé selon la revendication 7, caractérisé en ce que l'hybridation est réalisée à une température d'hybridation allant de 42°C à 60°C, avec du SSC 5-6 X et que, le lavage est réalisé ensuite entre 65°C et 75°C, avec du SSC 0,1 - 0,5 X.

**9.** Protéine présentant une homologie d'au moins 80 % avec la séquence d'acides aminés I, II ou III du tableau III.

**10.** Protéine selon la revendication 9 avec la séquence d'acides aminés de la protéine I, II ou III.

**11.** Utilisation des protéines selon les revendications 9 ou 10 pour l'obtention d'anticorps dirigés contre les bactéries du genre Listeria.

**12.** Anticorps pouvant être obtenus par immunisation d'animaux de laboratoire avec une protéine selon les revendications 9 ou 10.

**13.** Utilisation d'anticorps selon la revendication 12 pour la détermination immunologique des bactéries du genre Listeria.